# EUROPÄISCHE PATENTSCHRIFT

(11) **EP 3 539 475 B1**
(45) Veröffentlichungstag und Bekanntmachung des Hinweises auf die Patenterteilung: **21.07.2021**
(21) Anmeldenummer: 19158299.8
(22) Anmeldetag: 20.02.2019
(51) Int. Cl.: A61B 6/00

(54) **VERFAHREN ZUR BEWEGUNGSKORREKTUR VON SPEKTRALEN COMPUTERTOMOGRAPHIEDATEN, SOWIE EIN ENERGIESENSITIVES COMPUTERTOMOGRAPHIEGERÄT**
METHOD OF MOTION CORRECTION OF SPECTRAL COMPUTED TOMOGRAPHY DATA, AND AN ENERGY-SENSITIVE COMPUTER TOMOGRAPHY UNIT
PROCÉDÉ DE CORRECTION DE MOUVEMENT DES DONNÉES SPECTRALES DE TOMOGRAPHIE ASSISTÉE PAR ORDINATEUR AINSI QU'APPAREIL DE TOMOGRAPHIE ASSISTÉE PAR ORDINATEUR ÉNERGÉTIQUEMENT SENSIBLE

(30) Priorität: 16.03.2018 DE 102018204093
(43) Veröffentlichungstag der Anmeldung: 18.09.2019
(73) Patentinhaber: Siemens Healthcare GmbH, 91052 Erlangen (DE)
(72) Erfinder: Fournié, Eric, 91054 Erlangen (DE); Kappler, Steffen, 91090 Effeltrich (DE)

(56) Entgegenhaltungen:
- WO-A1-2017/216248
- L BOUSSEL ET AL: "Photon counting spectral CT component analysis of coronary artery atherosclerotic plaque samples", BRITISH JOURNAL OF RADIOLOGY., Bd. 87, Nr. 1040, 2. August 2014 (2014-08-02), Seite 20130798, XP055325294, GB ISSN: 0007-1285, DOI: 10.1259/bjr.20130798
- ALLEC N ET AL: "Paper;Including the effect of motion artifacts in noise and performance analysis of dual-energy contrast-enhanced mammography;Including the effect of motion artifacts in noise and performance analysis of dual-energy contrast-enhanced mammography", PHYSICS IN MEDICINE AND BIOLOGY, INSTITUTE OF PHYSICS PUBLISHING, BRISTOL GB, Bd. 57, Nr. 24, 30. November 2012 (2012-11-30), Seiten 8405-8425, XP020233404, ISSN: 0031-9155, DOI: 10.1088/0031-9155/57/24/8405
- Mathias Unberath: "Signal Processing for Interventional X-ray-based Coronary Angiography", Thesis- der Technischen Fakultat der Friedrich-Alexander-Universitat, 19 June 2017 (2017-06-19), XP055583251,
- HAHN JULIANE ET AL: "Motion compensation in the region of the coronary arteries based on partial angle reconstructions from short-scan CT data", MEDICAL PHYSICS., [Online] vol. 44, no. 11, 22 September 2017 (2017-09-22), pages 5795-5813, XP055779815, US ISSN: 0094-2405, DOI: 10.1002/mp.12514 Retrieved from the Internet: URL:https://api.wiley.com/onlinelibrary/td m/v1/articles/10.1002%2Fmp.12514> [retrieved on 2021-02-25]

## Beschreibung

Die Erfindung betrifft ein Verfahren zur Bewegungskorrektur von spektralen Computertomographiedaten, sowie ein energiesensitives Computertomographiegerät, mit dem bewegungskorrigierte CT-Bilder erzeugt werden können.

Computertomographie-Bildgebung wird in der klinischen Routine oft zur Darstellung von Herz-Kreislauf-Erkrankungen verwendet, da die Computertomographie (CT) vor allem bei Verwendung von jodhaltigem Kontrastmittel eine hochaufgelöste und kontrastreiche Darstellung von Blutgefäßen erlaubt. Auch für die Darstellung der Herzkrankgefäße wird Computertomographie verwendet, allerdings stößt die CT hier aufgrund der schnellen Herzbewegung an ihre Grenzen: Selbst bei Verwendung eines EKGs und einer entsprechenden Taktung der CT-Aufnahmen auf die Herzphasen mit geringerer Bewegung (z. B. der Diastole) ist es derzeit nicht möglich, einen vollständigen CT-Scan in so kurzer Zeit auszuführen, dass die Bewegung des Herzens keine Rolle spielt.

Aus diesem Grund wurden in der Vergangenheit eine Reihe von Methoden zur Bewegungskorrektur entwickelt, wie beispielhaft in den folgenden Veröffentlichungen beschrieben:
R. Bhagalia, J.D. Pack, and J.V. Miller. Nonrigid registration-based coronary artery motion correction for cardiac computed tomography. Med. Phys., Juli 2012.
J. Hahn, H. Bruder, T. Allmendinger, K. Stierstorfer, T. Flohr, and M. Kachelrieß. Cardiac Motion Compensation from Short-Scan CT Data: A comparison of Three algorithms. In Proceedings of the 4th International Conference on Image Formation in X-Ray computed Tomography, Bamberg, 2016. CT Meeting.
J. Hahn, H. Bruder, T. Allmendinger, K. Stierstorfer, T. Flohr, and M. Kachelrieß. Reduction of Motion Artifacts in Cardiac CT based on Partial Angle Reconstructions from Short Scan Data. In Medical Imaging, San Dieg, 2016. SPIE.
A.A. Isola, C.T. Metz, M. Schaap, S. Klein, M. Grass, and W.J. Niessen. Cardiac motion-corrected iterative cone-beam CT reconstruction using a semi-automatic minimum cost path-based coronary centerline extraction. Computerized Medical Imaging and Graphics, 2012.
S. Kim, Y. Chang and J.B. Ra. Cardiac motion correction based on partial angle reconstructed images in x-ray CT. Med. Phys., Mai 2015.
J.D. Pack, P.M. Edic, B.E.H. Claus, M. Iatrou, and J.V. Miller. Method for computed tomography motion estimation and compensation, Juli 2012. US Patent 8,224,056.
C. Rohkohl, H. Bruder, and K. Stierstorfer. Improving best-phase image quality in cardiac CT by motion correction with MAM optimization. Med. Phys., March 2013.
Q. Tang, J. Cammin, S. Srivastava, and K. Taguchi. A fully four-dimensional, iterative motion estimation and compensation method for cardiac CT. Med. Phys., Juli 2012.
Q. Tang, J. Matthews, M. Razeto, J.J. Linde, and S. Nakanishi. Motion estimation and compensation for coronary artery and myocardium in cardiac CT. In Medical imaging, San Diego, 2015. SPIE
U. van Stevendaal, J. von Berg, and M. Grass. A motion-compensated scheme for helical cone-beam reconstruction in angiography. Med. Phys., Juli 2007.
Der Übergang zwischen mehreren Stapeln aus Bildern wird in folgenden Schriften diskutiert:
B. Avants, C. Epstein, M. Grossmann and J. Gee. Symmetric diffeomorphic image registration with crosscorrelation: Evaluating automated labeling of elderly and neurodegenerative brain; Medical Image Analysis, 2008
A. Sotiras and N. Paragios. Discrete Symmetrie Image Registration, In 2012 9th IEEE International Symposium on Biomedical Imaging (ISBI), 2012.
T. Vercauteren, X. Pennec, A. Perchant and N. Ayache. Diffeomorphic demons: Efficient non-parametric image registration, NeuroImage, 2009.

Die meisten dieser Bewegungskorrekturalgorithmen haben gemeinsam, dass die Bewegung einer bestimmten Struktur in dem sich bewegenden Objekt, beispielsweise der kontrastmittelgefüllten Gefäße, verfolgt wird und daraus ein Bewegungsvektor erstellt wird. Dies setzt jedoch voraus, dass bestimmte, genau definierte Strukturen im bewegungsverzerrten Bild besonders gut zu erkennen und von ihrer Umgebung zu isolieren sind.
Darüber hinaus ist es oft notwendig, einen so großen Untersuchungsbereich aufzunehmen, dass dieser in mehrere Stapel aus Axialbildern aufgeteilt wird. Hierbei kann es gegebenenfalls zu Problemen durch Bewegungsartefakte kommen, d. h. die Stapel können nicht korrekt zusammengesetzt werden, weil der Patient sich zwischen der Aufnahme der einzelnen Stapel bewegt hat.
Manche CT-Geräte erlauben, energiesensitive Computertomographie(CT)-Daten aufzunehmen, d. h. das CT-Bild zeigt lediglich die Adsorption bzw. Schwächung von Photonen in einem bestimmten Energiebereich, im Folgenden auch Energieniveau genannt. Da verschiedene Strukturen im Körper bei verschiedenen Photonenenergien unterschiedliche Massen-Schwächungskoeffizienten aufweisen, können in durch spektrales CT in einer Aufnahme mehrerer Bilder des gleichen Untersuchungsbereichs mit unterschiedlichem Kontrast aufgenommen werden.
WO 2017/216248 A1 beschreibt ein energiesensitives CT-Gerät. Bei diesem werden Kalzium-Strukturen separiert, um einen Kalzium-Gehalt zu quantifizieren.
"Signal Processing for Interventional X-ray-based Coronary Angiography" von M. Unberath (Thesis- der Technischen Fakultät der Friedrich-Alexander-Universität, 19.Juni 2017 (2017-06-19), XP055583251) beschreibt Methoden der Atembewegungskompensation umfassend die Schätzung eines 3D Verschiebungsfelds aus rekonstruierten Bildern.
"Motion compensation in the region of the coronary arteries based on partial angle reconstructions from short-scan CT" von J.Hahn et al in Med.Phys 44(11), November 2017, beschreibt die Möglichkeit der Partial Angle Motion Compensation (PAMoCo).
Die Erfindung hat sich zur Aufgabe gesetzt, ein verbessertes Verfahren zur Bewegungskorrektur von Computertomographiebildern bereitzustellen. Insbesondere soll das Verfahren die Möglichkeit zur Bewegungskorrektur von CT-Bildern im Herzen verbessern.

Diese Aufgabe wird gelöst mit einem Verfahren gemäß Anspruch 1 und einem CT-Gerät gemäß Anspruch 10. Vorteilhafte Ausgestaltungen der Erfindung sind in den abhängigen Ansprüchen angegeben.

Die Erfindung beruht auf der Erkenntnis, dass es mit spektralen CT-Daten, d. h. CT-Daten, die nur ein bestimmtes Energieniveau bzw. einen bestimmten Energiebereich abdecken, möglich ist, bewegte Strukturen besonders einfach in den CT-Daten zu identifizieren und zur Bewegungskorrektur zu verwenden. Beispielsweise können die CT-Daten besonders sicher und ohne bzw. mit äußerst geringem Berechnungsaufwand in die verschiedenen Materialien zerlegt werden (Materialzerlegung oder engl. "Material Decomposition"). Dabei können beispielsweise jodgefüllte Gefäße oder Elemente des Skeletts identifiziert und ggf. extrahiert werden, die eine eventuelle Bewegung des Patienten bzw. des bewegten Organs anzeigen. Dadurch können die im Stand der Technik beschriebenen Bewegungskorrekturverfahren erheblich vereinfacht werden, denn eine aufwändige und fehleranfällige Segmentierung der Bewegungs-verunreinigten Bildvolumen, um derartige Strukturen zu isolieren, ist nicht mehr notwendig.

Das Verfahren umfasst die Bereitstellung von spektralen CT-Daten eines Untersuchungsbereiches mit einem sich bewegenden Objekt, wobei die spektralen CT-Daten mit einem energiesensitiven CT-Gerät aufgenommen wurden. Bei dem energiesensitiven CT-Gerät kann es sich um jegliches derzeit oder zukünftig verfügbares CT-Gerät handeln, welches es erlaubt, CT-Daten eines oder mehrerer bestimmter Energieniveaus getrennt voneinander aufzunehmen. Beispielsweise gibt es Dual-Energy- oder auch Dual-Source- oder Fast-Switching-Technologien, wie weiter unten noch ausgeführt.

Ein Untersuchungsbereich ist z.B. das Field-of-View einer CT-Untersuchung, insbesondere ein Ausschnitt des menschlichen oder tierischen Körpers. Bei dem sich bewegenden Objekt kann es sich um das menschliche Herz, jedoch auch um den Brustkorb oder um jedes andere Organ bzw. Teil des menschlichen oder tierischen Körpers handeln, denn jedes Körperteil kann während der CT-Messung bewegt werden und dadurch Bewegungsartefakte auslösen. Die Struktur ist bevorzugt ein Teil des sich bewegenden Objekts und bewegt sich mit, wie beispielsweise Blutgefäße in einem Organ oder Knochen oder Skelettelemente im menschlichen Körper allgemein. Vorzugsweise erstreckt sich die Struktur mit zumindest teilweise länglichen Erstreckungen durch das sich bewegende Objekt, insbesondere durch das darzustellende Organ. Insbesondere ist die Struktur ein Gefäß, der Schädel, Knochen, wie beispielsweise die Rippen, oder dergleichen.

Die CT-Daten enthalten zumindest ein Energieniveau, von denen zumindest ein Energieniveau an eine Struktur im sich bewegenden Objekt angepasst ist. Angepasst bedeutet, dass das Energieniveau bzw. mehrere Energieniveaus, bei denen CT-Daten aufgenommen werden, so ausgewählt ist, dass sich die Struktur auf den CT-Daten durch einen hohen Kontrast vom umliegenden Gewebe abhebt und somit leicht identifiziert werden kann. Falls die Struktur von Jod-kontrastierten Blutgefäßen gebildet wird, ist beispielsweise ein Energieniveau von ungefähr 33 keV geeignet, da der Massen-Schwächungskoeffizient von Jod hier ein Maximum aufweist, während Wasser relativ wenig absorbiert. Daher weisen die Gefäße bei dieser Energie einen extrem hohen Kontrast gegenüber dem umgebenden Gewebe auf.

In einem nächsten Schritt wird die Struktur in den CT-Daten dieses Energieniveaus identifiziert, wie weiter unten noch näher beschrieben. Insbesondere wird die Struktur zeitaufgelöst identifiziert, d. h. die sich während der CT-Aufnahme bewegende Struktur wird zumindest an einer, insbesondere an mehreren Positionspunkten, nachverfolgt. Daraus wird ein sogenanntes Bewegungsvektorfeld berechnet. Ein dreidimensionales Bewegungsvektorfeld stellt die Bewegung der Struktur zu einem bestimmten Zeitpunkt während der Aufnahme der CT-Daten dar, vorzugsweise jeweils an einem oder mehreren Positionspunkten innerhalb der Struktur. Insbesondere wird dieses Bewegungsvektorfeld für mehrere Zeitpunkte während der Aufnahmezeit der CT-Daten erstellt, sodass es sich um ein vierdimensionales Bewegungsvektorfeld handelt (die vierte Dimension ist die Zeit).

Dieses Bewegungsvektorfeld wird daraufhin dazu verwendet, eine Bewegungskorrektur der aufgenommenen CT-Daten durchzuführen und somit ein bewegungskorrigiertes CT-Bild zu berechnen. Im Vergleich zu der bisher durchgeführten algorithmischen Segmentierung bietet die hier mögliche Material-Zerlegung (Material Decomposition) durch spektrale CT-Aufnahmen den Vorteil, dass kein oder ein nur sehr geringer Berechnungsaufwand notwendig ist und somit die Zeit bis zum Erhalt des bewegungskorrigierten Bildes verringert wird. Ferner kann die Struktur besonders robust isoliert werden; die Gefahr von Fehlern bei der Segmentierung der Struktur im sich bewegenden Objekt ist verringert. Die in der Erfindung bevorzugt verwendete Material-Zerlegung basiert auf der spektralen Reaktion eines bestimmten chemischen Elementes, welches naturgegeben ist und somit nicht der Fehleranfälligkeit von Segmentierungsalgorithmen unterliegt, wie sie im Stand der Technik notwendig sind.

Durch die hiermit bereitgestellte robuste Bewegungskorrektur können CT-Aufnahmen nunmehr mit längeren Aufnahmezeiten oder langsameren Rotationsgeschwindigkeiten durchgeführt werden, wenn dies notwendig ist. Ferner kann durch die robuste Bewegungskorrektur auf Sedierung oder gar Narkose bei nicht kooperativen Patienten verzichtet werden. Schließlich werden auch eventuelle Wiederholungsscans vermieden, sodass die vom Patienten empfangene Röntgendosis reduziert wird.

Die Struktur wird vorzugsweise durch Materialzerlegung identifiziert. Dieses Verfahren beruht darauf, aus den spektralen CT-Daten Bilder zu berechnen, die die räumliche Verteilung der Materialdichte von zwei oder mehr Materialien darstellen. Wenn eines der Materialen nur in der Struktur vorkommt (z.B. Jod oder Kalzium), kann diese praktisch ohne weitere Bearbeitungsschritte identifiziert und ggf. aus den CT-Daten extrahiert werden. In einer Ausführungsform kann im Rahmen der Materialzerlegung eine Segmentierung, insbesondere mit einem Grenzwert, durchgeführt und die Struktur dadurch aus den CT-Daten extrahiert werden.

Gemäß einer bevorzugten Ausführungsform wird die Struktur bereits in den Sinogrammen der CT-Daten, also den Projektionsdaten, von dem/den an die Struktur angepassten Energieniveau(s) identifiziert. Damit kann die Struktur, beispielsweise das Blutgefäß, bereits auf dem Sinogramm vom umgebenden Gewebe isoliert werden. Dadurch werden Fehler bei der nachfolgenden Bildrekonstruktion vermieden. Die Identifizierung ergibt sich praktisch von selbst, da die Struktur auf den CT-Daten mit dem an die Struktur angepassten Energieniveau einen erheblich stärkeren Kontrast als das umgebende Gewebe aufweist. Gegebenenfalls kann noch eine leichte Grenzwert-Segmentierung angewendet werden, um das umliegende Gewebe mit Grauwerten unterhalb des Grenzwerts - z.B. im Bereich des Rauschens - sicher zu entfernen.

Gemäß einer Ausführungsform wird die Struktur aus den Sinogrammen der CT-Daten von einem einzigen an die Struktur angepassten Energieniveau extrahiert. Beispielsweise ist das an die Struktur angepasste Energieniveau ein solches, bei dem die Struktur die Photonen besonders stark absorbiert bzw. schwächt, sodass sich bereits mit den CT-Daten bei einem einzigen Energieniveau hervorragender Kontrast zwischen der Struktur und dem umliegenden Gewebe ergibt.

In einer anderen Ausführungsform wird die Struktur durch gewichtete Subtraktion der CT-Daten von zwei verschiedenen Energieniveaus extrahiert. Dies kann beispielsweise für Kalzium relevant sein, da Kalzium kein Maximum in seinem Massen-Schwächungskoeffizienten aufweist. Der Unterschied in der Abschwächungsrate von Kalzium und Wasser ist jedoch bei verschiedenen Energieniveaus unterschiedlich. Dieser Effekt kann dazu genutzt werden, durch eine gezielt gewichtete Subtraktion der CT-Daten bei verschiedenen Energieniveaus den Kontrast zwischen Kalzium und Wasser und somit den Kontrast zwischen Knochen und dem umgebenden wasserhaltigen weicheren Gewebe stark zu erhöhen. Gegebenenfalls kann anschließend noch eine Grenzwert-Segmentierung durchgeführt werden, um das umgebende Gewebe gänzlich zu entfernen. Auch für die gewichtet Subtraktion werden bevorzugt die Sinogramme verwendet, es können ggf. jedoch auch die rekonstruierten Bilddaten verwendet werden.

Aus den so erhaltenen CT-Daten, bevorzugt aus den Sinogrammen, welche nur noch die bewegte Struktur enthalten, kann ein Bewegungsvektorfeld berechnet werden.

Erfindungsgemäß wird aus den CT-Daten von dem/den an die Struktur angepassten Energieniveau(s) zumindest eine Zeitreihe von Partialbildern rekonstruiert und aus der/den Zeitreihen(n) von Partialbildern das Bewegungsvektorfeld berechnet. Bei einer möglichen Ausführungsform wird das Bewegungsvektorfeld wie folgt berechnet: Aus den CT-Daten von dem an die Struktur angepassten Energieniveau, insbesondere aus den CT-Daten, welche lediglich noch die identifizierte Struktur enthalten, wird durch Partial-Angle-Reconstruction zumindest eine Zeitreihe von Partialbildern rekonstruiert und daraus ein insbesondere vierdimensionales Bewegungsvektorfeld berechnet. Partial-Angle-Reconstruction bedeutet, dass nicht die Sinogrammdaten aus dem eigentlich notwendigen Winkelbereich von 180° + Fächerwinkel verwendet werden, sondern nur die Sinogramme aus einem kleinen Winkelsegment. Die Bildrekonstruktion ist aus diesem Grund fehleranfälliger, was jedoch durch die Reduzierung des Bildinhalts lediglich auf die identifizierte Struktur kompensiert wird. Die so erhaltenen Teilbilder haben eine gute zeitliche Auflösung, da sie naturgemäß in einem kleineren Zeitraum aufgenommen wurden als ein kompletter Scan von 180° + Fächerwinkel gebraucht hätte. Auf der so erzeugten Zeitreihe von Partialbildern kann die Struktur zum Beispiel an bestimmten festgelegten Landmarks oder Positionspunkten von einem Bild der Zeitreihe zum nächsten getrackt werden, d. h. ein bestimmter Positionspunkt wird auf der Zeitreihe von Bildern verfolgt. Derartige Verfahren werden als Feature-Tracking Verfahren bezeichnet. Aus der Verschiebung des jeweiligen Positionspunktes von einem Bild zum nächsten und dem bekannten zeitlichen Abstand zwischen den einzelnen Partialbildern kann dann für jedes Partialbild der Zeitreihe ein Bewegungsvektorfeld berechnet werden. Dadurch entsteht ein vierdimensionales Bewegungsvektorfeld. Die Dichte des Feldes im Raum kann den jeweiligen Bedürfnissen angepasst werden, bei Koronararterien kann beispielsweise alle 2 - 10 mm entlang der Koronararterie ein Positionspunkt gesetzt werden. Die Zeitauflösung der Zeitreihe von Partialbildern beträgt beispielsweise 5 - 50, insbesondere 8 - 20 ms. Falls die Struktur Blutgefäße umfasst, wird gemäß einer vorteilhaften Ausführungsform jeweils eine Mittellinie (Centerline) durch jedes Blutgefäß bestimmt.

Die obige Passage beschreibt das sogenannten PAMoCo-Verfahren (Motion Compensation based on Partial Angle Reconstructions), das erfindungsgemäße Verfahren kann jedoch auch auf jeglichen anderen Algorithmus zur Bewegungskompensation vorteilhaft angewendet werden.

In einer bevorzugten Ausführungsform werden die CT-Daten von dem an die Struktur angepassten Energieniveau in mehrere Stapel von Axialbildern aufgeteilt, wobei die Stapel mithilfe der identifizierten Struktur miteinander registriert werden. Dies hat den Vorteil, dass jeder Stapel unabhängig voneinander bearbeitet werden kann, wodurch Bewegungsartefakte auf die jeweiligen Bewegungen während der Akquisition eines Stapels reduziert werden. Bevorzugt überlappen sich die Stapel jeweils leicht in Axialrichtung. Gemäß einer Ausführungsform werden für die Rekonstruktion von jedem Stapel nur Kurz-Scandaten benutzt, die während eines Herzschlags akquiriert wurden.

Die Stapel werden bevorzugt mithilfe der identifizierten und ggf. extrahierten Struktur miteinander registriert. Die Aufnahme von CT-Daten von dem an die Struktur angepassten Energieniveau erlaubt eine einfache Isolation der Struktur und Material-Zerlegung und somit ein vereinfachtes Bild mit allgemein wenig Bildinhalt. Dies erleichtert die Registrierung. Dadurch können aneinander angrenzende Stapel robuster und einfacher miteinander registriert werden, das Risiko einer falschen Registrierung sinkt. Dies erleichtert zum einen die Bewegungskorrektur selbst. Darüber hinaus ermöglicht die Erfindung dadurch die Aufnahme von großen kontinuierlichen Volumen, da in der Z-Richtung größere Bildvolumina aufgenommen werden können. Selbst wenn der Patient sich während der Aufnahme (beispielsweise in einem Spiral-CT) bewegt, kann durch die Zerlegung des großen Aufnahmevolumens in einzelne Stapel und entsprechende erfindungsgemäße Verarbeitung der einzelnen Stapel und Registrierung angrenzender Stapel mithilfe der extrahierten Struktur ein großes Bildvolumen ohne Bewegungsartefakte erreicht werden. Dies erlaubt die Aufnahme von großen durchgängigen Volumen, die für rein diagnostische Zwecke besser geeignet sind.

Erfindungsgemäß wird aus den CT-Daten von dem/den an die Struktur angepassten Energieniveau(s) ein nicht-bewegungskompensiertes Bild rekonstruiert und auf diesem Bild eine Interessierende Region festgelegt, welche die Struktur oder einen Teil der Struktur einschließt, wobei die erfindungsgemäß rekonstruierte Zeitreihe von Partialbilder lediglich aus den zu dem Interessierenden Bereich gehörigen CT-Daten rekonstruiert wird. Die Rekonstruktion der Partialbilder kann dadurch erleichtert werden, dass nicht die Sinogrammdaten des gesamten Untersuchungsbereichs verwendet werden. Stattdessen werden die CT-Daten des an die Struktur angepassten Energieniveaus zunächst vollständig zu einem nichtbewegungskompensierten Bild rekonstruiert. Trotz Bewegungsartefakte sollte die Struktur auf diesem Bild einigermaßen erkennbar sein, insbesondere wenn es sich um kontrastierte Koronararterien handelt. Daraufhin wird um die Struktur herum ein Interessierender Bereich (Region of Interest - ROI) festgelegt, im Fall einer Koronararterie beispielsweise ein um die Mittellinie der Arterie verlaufender Schlauch. Diese interessierende Region sollte alle Bewegungsartefakte der Struktur umschließen. Die Partial-Angle-Reconstruction wird daraufhin lediglich für diesen Interessierenden Bereich durchgeführt; nicht zu diesem Bereich gehörende Sinogrammdaten werden ignoriert. Dadurch wird die Bildqualität der Zeitreihe von Partialbildern nochmals verbessert. Es können auch mehrere Interessierende Bereiche für verschiedene Teile der Struktur festgelegt werden.

Gemäß einer bevorzugten Ausführungsform wird für jeden Stapel von Axialbildern eine eigene Interessierende Region definiert, indem Interessierende Region, in welcher die Bewegungsartefakte enthalten sind, um die Struktur gelegt wird. Daraufhin wird für jeden Stapel jeweils die Interessierende Region durch Partial-Angle-Reconstruction zu einer Zeitserie von Partialbildern rekonstruiert, wobei die außerhalb der Interessierenden Region liegenden Sinogrammdaten ignoriert werden.

Die obige Passage beschreibt das sogenannte PAMoCo-Verfahren (Motion Compensation based on Partial Angle Reconstructions), das erfindungsgemäße Verfahren kann jedoch auch auf jeglichen anderen Algorithmus zur Bewegungskompensation vorteilhaft angewendet werden. Insbesondere kann eine Partial-Angle-Reconstruction auch für die gesamten Sinogrammdaten durchgeführt werden.

Die spektralen CT-Daten können CT-Daten von mehreren Energieniveaus enthalten. Das Bewegungsvektorfeld kann dabei zur Bewegungskorrektur der CT-Daten von dem an die Struktur angepassten Energieniveau oder von einem anderen Energieniveau verwendet werden. Die erstere Lösung ist besonders vorteilhaft bei angiographischen Anwendungen, bei denen nur Gefäße untersucht werden sollen. Die zweite Anwendung ist beispielsweise dann vorteilhaft, wenn eigentlich das Weichgewebe untersucht werden soll, die darin enthaltenen Skelettanteile jedoch die Struktur bilden, anhand derer die Bewegungskorrektur erfolgt.

Die Aufnahme der spektralen CT-Daten des Untersuchungsbereichs mit dem sich möglicherweise bewegenden Objekt ist in einigen Ausführungsformen Teil des Verfahrens. Das Verfahren kann jedoch auch offline durchgeführt werden, insbesondere auf einem beliebigen Rechner, dem die aufgenommenen spektralen CT-Daten zur Verfügung gestellt werden.
Das Verfahren ist dazu ausgelegt, auf einem beliebigen Rechner durchgeführt zu werden, z.B. einem Computer, Workstation, Netzwerkrechner, mobilen Gerät oder in der Cloud. Zur Durchführung des Verfahrens ist typischerweise eine Recheneinheit notwendig, beispielsweise eine CPU, zur Rekonstruktion der Bilddaten und zur Berechnung des Bewegungsvektorfeldes etc., sowie ein Datenspeicher zum Speichern der spektralen CT-Daten und des Bewegungsfeldes. Ein derartiger Rechner kann Teil der Konsole eines CT-Geräts sein, kann jedoch auch unabhängig davon vorhanden sein.
In einigen Ausführungsformen ist es zweckmäßig, gleichzeitig mit der Aufnahme der spektralen CT-Daten des Untersuchungsbereichs ein Elektrokardiogramm (EKG) der untersuchten Patienten aufzunehmen, insbesondere wenn das sich bewegende Objekt das Herz ist. Dies erlaubt, eine Triggerung oder ein Gating der Aufnahme nach dem Herzschlag, insbesondere Daten aus bestimmten Herzphasen aufzuzeichnen bzw. sicherzustellen, dass in jeder Herzphase die notwendigen Aufnahmen erfolgt sind. Mögliche Anwendungen des erfindungsgemäßen Verfahrens sind beispielsweise: Computertomographie des Herzens und der Blutgefäße unter Verwendung von jodhaltigem Kontrastmittel zur Isolierung der Gefäße. Mögliche Anwendungen sind ferner Neuro-CT unter Verwendung von Kalzium, um den Schädel zu isolieren, pädiatrisches CT unter Verwendung von Kalzium, um das Skelett zu isolieren, CT des Brustkorbs und der Lungenfunktion unter Verwendung von Kalzium, um die Rippen zu lokalisieren etc.

In einer möglichen Ausführungsform, welche nicht Teil der Vorliegenden Erfindung ist, erfolgt die Bewegungskorrektur z.B. mit den folgenden Schritten:
1. Bildrekonstruktion unter Verwendung von Rückprojektionsalgorithmen der CT-Daten mit dem an die Struktur angepassten Energieniveau. Dadurch wird ein Bildvolumen mit Bewegungsartefakten erhalten.
2. Auf diesem Volumen wird die Struktur identifiziert. Im Stand der Technik war es hierzu notwendig, eine algorithmische Segmentierung durchzuführen, beispielsweise durch Grenzwert-Verfahren. Mit der Erfindung entfällt dieser Segmentierungsschritt in der Regel, da die bewegte Struktur bereits optimal sichtbar ist.
3. Schätzen der Bewegung durch Berechnen von Bewegungsvektorfeldern. Hierfür können verschiedene Methoden zum Einsatz kommen.
4. Bewegungskorrektur der CT-Daten durch das Bewegungsvektorfeld, entweder im Bildraum oder im Sinogrammraum.

Die Erfindung ist auch auf ein energiesensitives CT-Gerät gerichtet, welches dazu konfiguriert ist, das Verfahren nach einem der vorhergehenden Schritte auszuführen. Gemäß einer bevorzugten Ausführungsform weist das energiesensitive CT-Gerät einen Multi-Energie-Detektor auf, welcher gleichzeitig spektrale CT-Daten von mehreren verschiedenen Energieniveaus aufnehmen kann. Besonders bevorzugt ist ein sogenannter Photon-Counting-Detektor. Diese neue Technologie beruht auf besonderen Materialien, welche Röntgenstrahlen direkt in elektrische Impulse umwandeln, deren Spannung korreliert ist mit der Energie des eingehenden Photons. Dadurch ist es theoretisch möglich, einzeln Photonen zu zählen und somit ein komplettes Energiespektrum aufzunehmen. Das Verfahren kann aber auch mit den bereits jetzt verwendeten Dual-Energy-CTs durchgeführt werden, bei welchen Photonen auf zwei verschiedenen Energieniveaus ausgesandt und mit zwei separaten Detektoren aufgenommen werden. ES ist vorteilhaft, wenn das CT-Gerät erlaubt, gleichzeitig auf derselben Position CT-Daten mit verschiedenen Energien aufzunehmen. Daher sind Ausführungsformen von CT-Geräten, bei welchen zwei verschiedene Röntgenröhren/Detektor-Systeme um 90° versetzt betrieben werden, weniger bevorzugt.

Die Erfindung wird nun anhand von Ausführungsbeispielen mit Bezug auf die beiliegenden Zeichnungen näher beschrieben. In den Zeichnungen zeigen:
- Figur 1: eine erste Ausführungsform eines erfindungsgemäßen CT-Geräts;
- Figur 2: eine zweite Ausführungsform eines erfindungsgemäßen CT-Geräts;
- Figur 3: eine schematische Ansicht des Herzens mit segmentierten Koronararterien;
- Figur 4: eine schematische Ansicht des Herzens mit zwei Stapeln von Axialbildern;
- Figur 5: eine schematische Ansicht des Herzens mit eingezeichneter Interessierender Region um eine Koronararterie;
- Figur 6: Sinogramm-Daten der CT-Daten von dem an die Struktur angepassten Energieniveau, eingeteilt in Winkelabschnitte für Partial-Angle-Reconstruction;
- Figur 7: eine schematische Ansicht eines Herzens mit Bewegungsfeld;
- Figur 8: ein Flussdiagramm des erfindungsgemäßen Verfahrens.

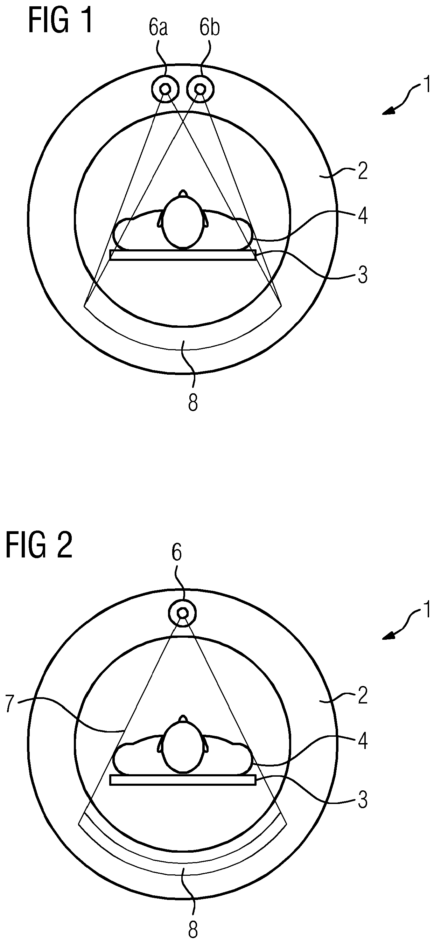

Figur 1 zeigt einen schematischen Querschnitt durch ein CT-Gerät 1, welches ein schnelles Wechseln zwischen zwei verschiedenen Energieniveaus erlaubt. In der Gantry 2 befindet sich ein Patiententisch 3 mit darauf liegendem Patienten 4. Zwei verschiedene Röntgenquellen 6a, 6b senden abwechselnd in schnellem Wechsel Röntgenstrahlen bei verschiedenen Energien aus, beispielsweise bei 80 und 140 keV, oder 33 und 80 keV.

Der Röntgendetektor 8 wird entsprechend geschaltet und ordnet die jeweils detektierten Photonen den verschiedenen Energieniveaus zu. Hier ist also nur ein Detektor notwendig, um mehrere Energieniveaus aufzunehmen.

Figur 2 zeigt eine andere Ausführungsform, bei welcher lediglich eine Röntgenquelle 6 vorhanden ist, welche Photonen auf verschiedenen Energieniveaus aussendet. Dafür ist der Detektor 8 zum Detektieren verschiedener Energieniveaus ausgelegt, beispielsweise durch die Split-Beam-Technologie, bei der das Spektrum in Hochenergie- und Niedrigenergiespektren aufgeteilt wird. Denkbar ist ebenfalls die Verwendung eines Photon-Counting-Detektors 8. Die von der Röntgenquelle 6 ausgesandten Röntgenstrahlen haben im Allgemeinen die Form eines Fächers 7. Es kann sich bei dem Röntgengerät 1 um ein Spiral-CT handeln.

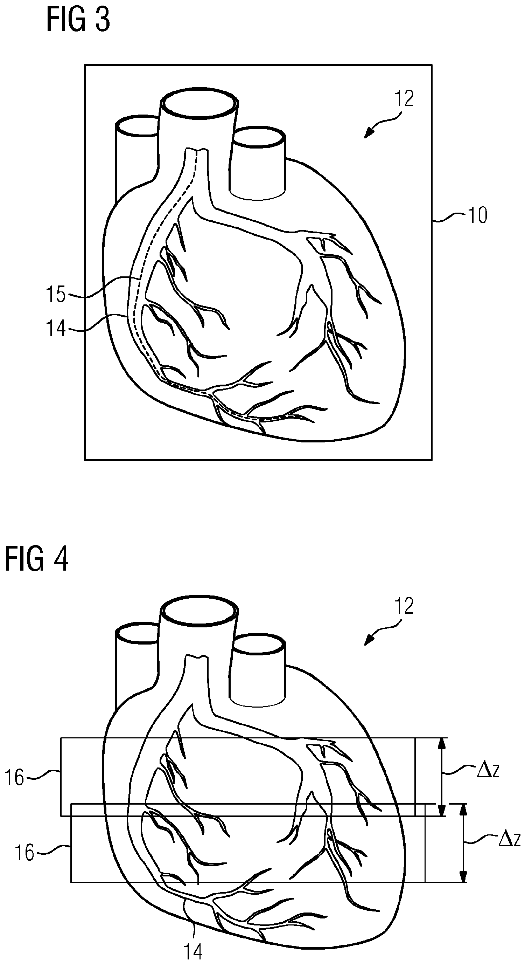

Figur 3 zeigt beispielhaft ein sich bewegendes Objekt 12, hier das menschliche Herz, in einem Untersuchungsbereich 10. Der Untersuchungsbereich 10 ist in der Regel das Field-of-View eines CT-Geräts, erstreckt sich also über eine größere Breite in Axialrichtung als auf dem Bild dargestellt. In der Herzwand verlaufen die Koronararterien 14. Werden diese mit jodhaltigem Kontrastmittel auf bekannte Art kontrastiert und gleichzeitig spektrale CT-Daten auf einem Energieniveau aufgenommen, bei dem Jod einen besonders hohen Massen-Schwächungskoeffizienten aufweist, können CT-Daten aufgenommen werden, die praktisch selektiv ausschließlich die Koronargefäße 14 zeigen.

Gemäß einer Ausführungsform ist es daher möglich, ein nichtbewegungsregiertes Bildvolumen aus den spektralen CT-Daten zu rekonstruieren und darauf die Koronararterien zu identifizieren oder zu segmentieren, wie hier durch Schraffur angezeigt. Ferner ist es auch möglich, eine Mittellinie 15 des Gefäßes zu bestimmen, die hier gestrichelt eingezeichnet ist. Idealerweise wird zur Segmentierung eine Short-Scan-Reconstruction des gesamten Volumens durchgeführt.

Wie in Figur 4 dargestellt, kann in einem nächsten Schritt das Volumen in mehrere überlappende Stapel 16 aufgeteilt werden, wobei jeder Stapel 16 die Ausdehnung Δz in der Längsrichtung des Geräts aufweist. Für die Rekonstruktion jedes Stapels werden bevorzugt nur kurze Scandaten verwendet, die während eines Herzschlags aufgenommen wurden. Bevorzugt werden die CT-Daten jedes Stapels unabhängig voneinander verarbeitet.

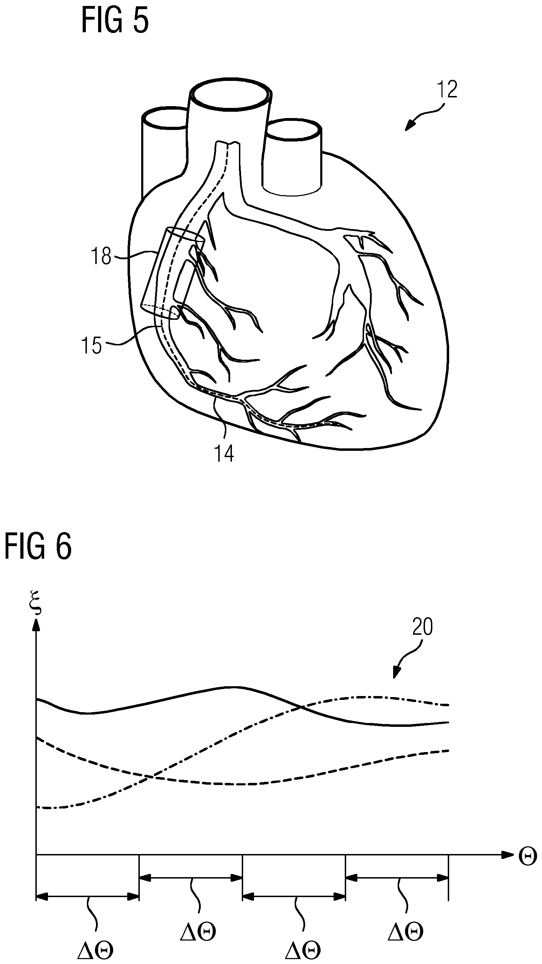

Insbesondere für jeden Stapel 16 kann in einem weiteren Schritt eine Interessierende Region 18 festgelegt werden, wie in Figur 5 gezeigt. Diese wird vorzugsweise im Fall der Koronararterien dadurch gebildet, dass ein Schlauch 18 mit einem bestimmten Radius um die bereits auf dem nichtbewegungskorrigierten Bild identifizierten Mittellinie 15 der Koronararterie 14 gelegt wird. Diese Region 18 sollte alle Bewegungsartefakte enthalten, die durch die Bewegung der Koronararterien 14 erzeugt werden. Der Radius des Schlauchs 18 kann gegebenenfalls vorab festgelegt sein.

Die Festlegung einer Interessierenden Region 18 ist optional, das Verfahren könnte auch das komplette Bildvolumen bearbeiten.

In einem nächsten Schritt wird vorzugsweise eine Zeitreihe von Partialbildern durch Partial-Angle-Reconstruction rekonstruiert. Dazu ist es hilfreich, wenn die Sinogrammdaten 20, wie in Figur 6 schematisch dargestellt, in eine Anzahl überlappender Sektoren Δθ aufgeteilt werden. Δθ steht für den Teilwinkel, und die Daten aus jedem Δθ werden zu einem Partialbild rekonstruiert. Hierzu ist es bevorzugt, wenn ausschließlich die Sinogrammdaten aus der Region von Interesse 18 verwendet werden. Aus den so konstruierten Partialbildern kann ein entsprechendes Bewegungsvektorfeld berechnet werden.

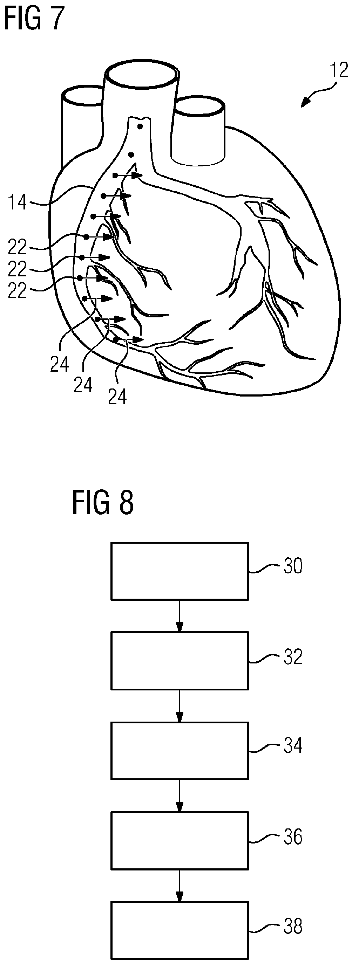

Figur 7 zeigt dazu eine Anzahl von Positionspunkten 22, die jeweils entlang der Koronararterie 14 angelegt werden. Jeder Positionspunkt 22 wird über die Zeitreihe der Partialbilder getrackt und darauf für jedes Partialbild, d. h. für jeden Punkt der Zeitreihe, ein Bewegungsvektor 24 ermittelt. Aus diesen Bewegungsvektoren ergibt sich das vierdimensionale Bewegungsvektorfeld.

Das Bewegungsvektorfeld wird dann dazu verwendet, entweder das in Figur 3 dargestellte unkorrigierte Bildvolumen des Herzens 12 oder aber die Sinogrammdaten entsprechend zu korrigieren, um ein bewegungskorrigiertes CT-Bild zu erhalten.

Figur 8 gibt noch mal einen Überblick über das erfindungsgemäße Verfahren. In einem Schritt 30 werden spektrale CT-Daten eines Untersuchungsbereichs 10 mit einem sich bewegenden Objekt 12 bereitgestellt oder aufgenommen, wobei diese CT-Daten von zumindest einem Energieniveau enthalten, welches an eine Struktur 14 im sich bewegenden Objekt 12 angepasst ist.
In Schritt 32 wird die Struktur in den CT-Daten identifiziert und ein oder mehrere Bildvolumen rekonstruiert, beispielsweise durch Partial-Angle-Reconstruction. In Schritt 34 wird hieraus ein Bewegungsvektorfeld berechnet. Dieses wird in Schritt 36 zur Bewegungskorrektur der spektralen CT-Daten verwendet, sodass in 38 ein bewegungskorrigiertes CT-Bild bereitgestellt werden kann.

## Patentansprüche

1. Verfahren zur Bewegungskorrektur von Computertomographiebildern, umfassend die folgenden Schritte:
a) Bereitstellen von spektralen CT-Daten (20) eines Untersuchungsbereichs (10) mit einem sich bewegenden Objekt (12), wobei die spektralen CT-Daten mit einem energiesensitiven CT-Gerät (1) aufgenommen wurden und CT-Daten von zumindest einem Energieniveau enthalten, und wobei zumindest ein Energieniveau der CT-Daten an eine Struktur (14) im sich bewegenden Objekt angepasst ist;
b) Identifizieren der Struktur (14) in den CT-Daten von dem/den an die Struktur angepassten Energieniveau(s);
c) Berechnen eines Bewegungsvektorfeldes (24) der identifizierten Struktur (14);
d) Bewegungskorrektur der spektralen CT-Daten durch das Bewegungsvektorfeld und Berechnung eines bewegungskorrigierten CT-Bildes, **dadurch gekennzeichnet, dass** aus den CT-Daten von dem/den an die Struktur (14) angepassten Energieniveau(s) zumindest eine Zeitreihe von Partialbildern rekonstruiert wird und aus der/den Zeitreihen(n) von Partialbildern das Bewegungsvektorfeld (24) berechnet wird, und
wobei aus den CT-Daten von dem/den an die Struktur angepassten Energieniveau(s) ein nicht-bewegungskompensiertes Bild rekonstruiert wird und auf diesem Bild eine Interessierende Region (18) festgelegt wird, welche die Struktur (14) oder einen Teil der Struktur einschließt, und wobei die Zeitreihe von Partialbilder lediglich aus den zu dem Interessierenden Bereich gehörigen CT-Daten rekonstruiert wird.

2. Verfahren nach Anspruch 1, wobei die Struktur (14) in den Sinogrammen (20) der CT-Daten von dem/den an die Struktur angepassten Energieniveau(s) identifiziert wird.

3. Verfahren nach Anspruch 1 oder 2, wobei die Struktur durch Materialzerlegung identifiziert wird.

4. Verfahren nach einem der vorhergehenden Ansprüche, wobei die Struktur (14) in den CT-Daten von einem einzigen an die Struktur angepassten Energieniveau identifiziert wird, und/oder wobei die Struktur durch gewichtete Subtraktion der CT-Daten von zwei verschiedenen Energieniveaus identifiziert wird.

5. Verfahren nach einem der vorhergehenden Ansprüche, wobei die CT-Daten in mehrere Stapel (16) von Axialbildern aufgeteilt werden, und wobei die Stapel mithilfe der identifizierten Struktur (14) miteinander registriert werden.

6. Verfahren nach einem der vorhergehenden Ansprüche, wobei die spektralen CT-Daten von mehreren Energieniveaus enthalten, und wobei das Bewegungsvektorfeld zur Bewegungskorrektur der CT-Daten von dem an die Struktur angepassten Energieniveau oder von einem anderen Energieniveau verwendet wird.

7. Verfahren nach einem der vorhergehenden Ansprüche 1 - 6, wobei die Struktur ein Jod-kontrastiertes Blutgefäß (14) ist, und insbesondere eine Koronararterie.

8. Verfahren nach einem der vorhergehenden Ansprüche 1 - 6, wobei die Struktur ein Teil des Skeletts einschließlich des Schädels ist und zumindest ein an die Struktur angepasstes Energieniveau in einem Bereich liegt, in dem Wasser und Kalzium stark voneinander abweichende Massen-Schwächungskoeffizienten aufweisen.

9. Verfahren nach einem der vorhergehenden Ansprüche, enthaltend den Schritt:
• Aufnehmen von spektralen CT-Daten des Untersuchungsbereichs mit dem sich bewegenden Objekt mit einem energiesensitiven CT-Gerät, wobei die spektralen CT-Daten CT-Daten von zumindest einem Energieniveau enthalten, und wobei zumindest ein Energieniveau an eine Struktur im sich bewegenden Objekt angepasst ist.

10. Energiesensitives CT-Gerät (1), welches dazu konfiguriert ist, das Verfahren nach einem der vorhergehenden Ansprüche auszuführen.

11. Energiesensitives CT-Gerät nach Anspruch 10, wobei das CT-Gerät einen Multi-Energie-Detektor (8) aufweist, welcher gleichzeitig spektrale CT-Daten von mehreren verschiedener Energieniveaus aufnehmen kann, insbesondere einen Photon-Counting-Detektor.

## Claims

1. Method for motion correction of computed tomography images comprising the following steps:
a) provision of spectral CT data (20) of an examination region (10) with a moving object (12), wherein the spectral CT data was recorded with an energy-sensitive CT device (1) and includes CT data at at least one energy level and wherein at least one energy level of the CT data is adapted to a structure (14) in the moving object;
b) identification of the structure (14) in the CT data at the energy level(s) adapted to the structure;
c) calculation of a motion vector field (24) of the identified structure (14);
d) motion correction of the spectral CT data by the motion vector field and calculation of a motion-corrected CT image,
**characterised in that** at least one time sequence of partial images is reconstructed from the CT data at the energy level(s) adapted to the structure (14) and the motion vector field (24) is calculated from the time sequence(s) of partial images, and
wherein a non-motion-compensated image is reconstructed from the CT data at the energy level(s) adapted to the structure and on this image a region of interest (18) is defined that includes the structure (14) or a part of the structure and wherein the time sequence of partial images is reconstructed solely from the CT data belonging to the region of interest.

2. Method according to claim 1, wherein the structure (14) is identified in the sinograms (20) of the CT data at the energy level(s) adapted to the structure.

3. Method according to claim 1 or 2, wherein the structure is identified by material decomposition.

4. Method according to one of the preceding claims, wherein the structure (14) in the CT data is identified at a single energy level adapted to the structure, and/or wherein the structure is identified by weighted subtraction of the CT data at two different energy levels.

5. Method according to one of the preceding claims, wherein the CT data is divided into several stacks (16) of axial images, and wherein the stacks are registered to one another with the aid of the identified structure (14).

6. Method according to one of the preceding claims, wherein the spectral CT data includes at several energy levels and wherein the motion vector field is used for motion correction of the CT data at the energy level adapted to the structure or at another energy level.

7. Method according to one of the preceding claims 1 - 6, wherein the structure is an iodine-contrasted blood vessel (14) and in particular a coronary artery.

8. Method according to one of the preceding claims 1 - 6, wherein the structure is a part of the skeleton including the skull and at least one energy level adapted to the structure lies in a region in which water and calcium have very different mass attenuation coefficients.

9. Method according to one of the preceding claims, including the step:
• recording spectral CT data of the examination region with the moving object with an energy-sensitive CT device, wherein the spectral CT data includes CT data at at least one energy level and wherein at least one energy level is adapted to a structure in the moving object.

10. Energy-sensitive CT device (1) configured to carry out the method according to one of the preceding claims.

11. Energy-sensitive CT device according to claim 10, wherein the CT device has a multi-energy detector (8) able to record spectral CT data at several different energy levels simultaneously, in particular a photon-counting detector.

## Revendications

1. Procédé de correction de mouvement d'images de tomodensitométrie assisté par ordinateur, comprenant les stades suivants :
a) on se procure des données (20) CT spectrales d'une partie (10) à examiner ayant un objet (12) en mouvement, les données CT spectrales ayant été enregistrées par un appareil (1) CT sensible à une énergie et contenant des données CT d'au moins un niveau d'énergie, et dans lequel au moins un niveau d'énergie des données CT est adapté à une structure (14) de l'objet en mouvement ;
b) on identifie la structure (14) dans les données CT à partir du/des niveau(x) d'énergie adapté à la structure;
c) on calcule un champ (24) de vecteur de mouvement de la structure (14) identifiée ;
d) on corrige en mouvement les données CT spectrales par le champ de vecteur de mouvement et on calcule une image CT corrigée en mouvement,
**caractérisé en ce que**
à partir des données CT du/des niveau(x) d'énergie adapté à la structure (14), on reconstruit au moins une série temporelle d'images partielles et à partir de la/des série(s) temporelle d'images partielles on calcule le champ (24) de vecteur de mouvement, et
dans lequel à partir des données CT du/des niveau(x) d'énergie adapté à la structure, on reconstruit une image non compensée en mouvement et on fixe sur cette image une région (18) à laquelle on s'intéresse qui enferme la structure (14) ou une partie de la structure et dans lequel on reconstruit la série temporelle d'images partielles seulement à partir des données CT appartenant à la région à laquelle on s'intéresse.

2. Procédé suivant la revendication 1, dans lequel on identifie la structure (14) dans les sinogrammes (20) des données CT à partir du/des niveau(x) d'énergie adapté (s) à la structure.

3. Procédé suivant la revendication 1 ou 2, dans lequel on identifie la structure par décomposition de matière.

4. Procédé suivant l'une des revendications précédentes, dans lequel on identifie la structure (14) dans les données CT à partir d'un niveau d'énergie unique adapté à la structure et/ou dans lequel on identifie la structure par soustraction pondérée des données CT de deux niveaux d'énergie différents.

5. Procédé suivant l'une des revendications précédentes, dans lequel on répartit les données CT en plusieurs piles (16) d'images axiales et dans lequel on repère entre elles les piles à l'aide de la structure identifiée.

6. Procédé suivant l'une des revendications précédentes, dans lequel les données CT spectrales proviennent de plusieurs niveaux d'énergie et dans lequel on utilise le champ de vecteur de mouvement pour la correction en mouvement des données CT par le niveau d'énergie adapté à la structure ou par un autre niveau d'énergie.

7. Procédé suivant l'une des revendications 1 à 6 précédentes, dans lequel la structure est un vaisseau (14) sanguin ayant subi une injection d'agent de contraste iodé, et notamment une artère coronarienne.

8. Procédé suivant l'une des revendications 1 à 6 précédentes, dans lequel la structure est une partie du squelette, y compris du crâne et au moins un niveau d'énergie adapté à la structure se trouve dans une partie dans laquelle l'eau et le calcium ont des coefficients d'affaiblissement massique, qui diffèrent beaucoup l'un de l'autre.

9. Procédé suivant l'une des revendications précédentes, comprenant le stade :
• enregistrement de données CT spectrales de la partie à examiner ayant l'objet en mouvement par un appareil CT sensible à l'énergie, dans lequel les données CT spectrales contiennent des données CT d'au moins un niveau d'énergie et dans lequel au moins un niveau d'énergie est adapté à une structure de l'objet en mouvement.

10. Appareil (1) CT sensible à l'énergie, qui est configuré pour effectuer un procédé suivant l'une des revendications précédentes.

11. Appareil CT sensible à l'énergie suivant la revendication 10, dans lequel l'appareil CT a un détecteur (8) de multiénergie, qui peut recevoir simultanément des données CT spectrales de plusieurs niveaux d'énergie différents, en étant notamment un compteur de photons.
